# EUROPÄISCHE PATENTSCHRIFT

(11) **EP 1 267 797 B1**
(45) Veröffentlichungstag und Bekanntmachung des Hinweises auf die Patenterteilung: **24.08.2005**
(21) Anmeldenummer: 01940285.8
(22) Anmeldetag: 04.04.2001
(51) Int. Cl.: A61K 6/083

(54) **ZWEIKOMPONENTIGE DENTALMASSEN MIT NIEDRIGER ABBINDETEMPERATUR**
TWO-COMPONENT DENTAL MATERIALS HAVING A LOW SETTING TEMPERATURE
PRODUITS DENTAIRES A DEUX COMPOSANTES ET A FAIBLE TEMPERATURE DE PRISE

(30) Priorität: 07.04.2000 DE 10017188
(43) Veröffentlichungstag der Anmeldung: 02.01.2003
(73) Patentinhaber: 3M ESPE AG, 82229 Seefeld (DE)
(72) Erfinder: LEHMANN, Thomas, 84489 Burghausen (DE); HECHT, Reinhold, D-86916 Kaufering (DE)
(74) Vertreter: Brem, Roland
(86) Internationale Anmeldenummer: PCT/EP2001/003834
(87) Internationale Veröffentlichungsnummer: WO 2001/076536

(56) Entgegenhaltungen:
- EP-A- 0 059 451
- EP-A- 0 374 824
- DE-A- 19 928 238

## Beschreibung

Die vorliegende Erfindung betrifft zweikomponentige radikalisch polymerisierbare Dentalmassen, die bei guter mechanischer Endhärte eine geringe Temperaturerhöhung bei der Abbindung aufweisen.

Üblicherweise bestehen solche Dentalmassen aus einer Monomermischung, einem Initiatorsystem, Füllstoffen, Additiven und gegebenenfalls Weichmachern.

Als Monomere in polymerisierbaren Dentalmassen werden vor allem ungesättigte Verbindungen, wie Acrylsäure- und/oder Methacrylsäureester, verwendet.

Bei der Anwendung dieser Dentalmassen werden die Monomere mit einem geeigneten Initiatorsystemen vermischt, wobei eine pastöse Masse entsteht, die durch radikalische Polymerisation aushärtet.

Als Auslöser der radikalischen Polymerisation werden verschiedene Redoxinitiatorsysteme eingesetzt.

Ein bereits lange bekanntes Initiatorsystem besteht aus einer Amin- und einer Peroxidkomponente, wie in DT-PS-9 75 072 beschrieben. Die Polymerisation wird hierbei durch die Peroxidverbindung gestartet. Als Beschleuniger der Polymerisation wird beispielsweise ein tertiäres aromatisches Amin eingesetzt.

Ein weiteres solches System wird auch von Albert Gross in "Quintessenz der Zahntechnik", 1977, 7, Referat Nr. 293, beschrieben. Dort beschleunigen sekundäre oder tertiäre Amine den Zerfall der Peroxidkomponente, die die Polymerisation der Monomere auslöst. Üblicherweise wird die Amin-Komponente dabei in eine Paste, die sogenannte Basispaste, eingearbeitet. Diese Basispaste enthält auch die zur Polymerisation vorgesehenen Monomere. Die PeroxidKomponente wird in eine weitere Paste, die sogenannte Katalysatorpaste, eingearbeitet. Die räumliche Trennung der beiden Initiatorkomponenten ist erforderlich, um eine vorzeitige Aushärtung der Monomere zu vermeiden.

Auch in der deutschen Patentschrift DE-955 633 wird ein ähnliches Initiatorsystem für die Polymerisation von ungesättigten Kohlenwasserstoffen beschrieben, das Schwermetalle sowie eine Amin- und eine Sulfonkomponente enthält.

Auch in der EP-B-0 374 824 wird ein Initiatorsystem mit einer organischen Peroxidverbindung und einem tertiären aromatischen Amin als Aktivator (Beschleuniger) genannt.

Nachteilig an Dentalmassen mit diesem Initiatorsystem ist, dass die für eine günstige Abbindephase geeigneten aromatischen Amine zu Verfärbungen neigen. Diese gelb-braunen Farbveränderungen sind im dentalen Bereich aber nicht akzeptabel. Außerdem sind tertiäre aromatische Amine aufgrund ihrer Gesundheitsgefährdung nur bedingt einsetzbar. Weiterhin problematisch ist der Temperaturanstieg bei der Polymerisation dieser Systeme aufgrund der exothermen Prozesse. Eine zu hohe Wärmeentwicklung kann zu Pulpaschädigungen des Patienten führen.

Eine günstigere Temperaturentwicklung und auch bessere Farbstabilität weisen die Initiatorsysteme auf, die beispielsweise in der DT-C-14 95 520 beschrieben werden. Die Zusammensetzung hieraus polymerisiert mit einer relativ geringen Temperaturerhöhung in kurzer Zeit und ohne Zuführung von externer Energie. Die beschriebenen Systeme enthalten Barbitursäurederivate bzw. Malonylsulfamide, organische Peroxide, ionogen gebundenes Halogen und/oder eine Schwermetallverbindung.

Auch die EP-B-0 374 824 beschreibt ein derartiges Initiatorsystem aus Barbitursäurederivat, Peroxid, Schwermetallverbindung und ionogenem Halogen. Bei diesen Initiatorsystemen können Barbitursäurederivate bzw. Malonylsulfamide und Peroxide nicht gemeinsam gelagert werden. Ferner müssen auch beide genannten Bestandteile des Initiatorsystems von den Monomeren getrennt aufbewahrt werden. Es ist also zur Bereitstellung von polymerisierbaren Dentalmassen, die Monomere, Barbitursäurederivate bzw. Malonylsulfamide, organische Peroxide, ionogen gebundenes Halogen und/oder eine Schwermetallverbindung enthalten, eine Lagerung in drei räumlich voneinander getrennten Pasten notwendig.

Dies führt zu einer relativ aufwendigen Handhabung der Systeme. So sind dreikomponentige Systeme für eine automatische Anmischung nicht geeignet. Daher müssen diese herkömmlichen Dentalmassen aus drei Komponenten von Hand angemischt werden, wobei Luft eingearbeitet wird und die Dosierung der Einzelkomponenten nicht exakt erfolgen kann. Die Einarbeitung von Luft ist vor allem deshalb zu vermeiden, da durch Luftblasen Fehlstellen in dem ausgehärteten Material entstehen. Dadurch erhöht sich die Bruchempfindlichkeit und es resultiert eine schlechte Oberflächenbeschaffenheit. Unterschiedliche Dosierungen führen zu veränderten Abbindezeiten, verschlechterten mechanischen Eigenschaften und einer ungenauen Farbgebung. Weiterhin ist die Handanmischung zeitaufwendiger als eine automatische Mischung.

Die WO 00/78271 A1 betrifft eine polymerisierbare Dentalmasse, die u. a. ein bi- oder höherfunktionellen ethylenisch ungesättigtes Monomer, ein monofunktionelles ethylenisch ungesättigtes Monomer, einen Beschleuniger, Weichmacher und ein Redoxinitiatorsystem enthält, welches Barbitursäure und/oder Malonylsulfamid und ein organisches Peroxid umfasst. Die Masse kann lagerstabil in zwei räumlich von einander getrennten Pasten bereitgestellt werden.

Ferner werden in der DT-C-14 955 20 Methacrylatpolymerisationen in Lösungsmitteln beschrieben, die Barbitursäurederivate bzw. Malonylsulfamide, ionogen gebundenes Halogen und/oder eine Schwermetallverbindung enthalten und statt mit organischen Peroxiden mit Sauerstoff aushärten.

Versucht man allerdings diese Systeme lösungsmittelfrei in Kartuschen zu lagern, so findet zwar eine Aushärtung nach dem Anmischen statt, aber die mechanischen Werte des ausgehärteten Materials liegen niedriger als bei Zusatz von Peroxiden, die allerdings wiederum eine höhere Abbindetemperatur erzeugen und toxikologisch ungünstig sind.

Aufgabe dieser Erfindung ist es, eine Dentalmasse bereitzustellen, die basierend auf einem Initiatorsystem, enthaltend Barbitursäurederivat und/oder Malonylsulfamid, gegebenenfalls einem ionogen gebundenem Halogen, einer Schwermetallverbindung und Sauerstoff aushärtet und die Nachteile des Standes der Technik vermeidet.

Überraschenderweise hat sich gezeigt, dass durch Zusatz von Vinylethern zu Basispasten, basierend auf ethylenisch ungesättigten Monomeren, welche keine Vinylether sind, höhere mechanische Werte erzielt werden können und die Abbindetemperatur niedrig bleibt.

Die erfindungsgemäßen Massen bestehen aus zwei Komponenten, wobei Komponente I:
(a) 0,1 bis 20 Gew.-%, bevorzugt 0,5 bis 18 Gew.-% und besonders bevorzugt 1 bis 13 Gew.-% mindestens eines Vinylethers,
(b) 10 bis 89,9 Gew.-%, bevorzugt 24,5 bis 89 Gew.-% und besonders bevorzugt 30 bis 85 Gew.-% mindestens eines ethylenisch ungesättigten Monomers, welches kein Vinylether ist,
(c) 0,001 bis 5 Gew.-%, bevorzugt 0,002 bis 4 Gew.-% und besonders bevorzugt 0,005 bis 3 Gew.-% mindestens eines Beschleunigers,
(d) 9,999 bis 89,899 Gew.-%, bevorzugt 15 bis 85 Gew.-% und besonders bevorzugt 20 bis 70 Gew.-% Füllstoffe, Thixotropie-Hilfsmittel, Verzögerer und andere Hilfsstoffe,
   und Komponente II:
(e) 0,1 bis 20 Gew.-%, bevorzugt 1 bis 18 Gew.-% und besonders bevorzugt 3 bis 13 Gew.-% mindestens eines Barbitursäurederivates und/oder eines Malonylsulfamides, das die radikalische Polymerisation auslösen kann, und
(f) 0 bis 79,9 Gew.-%, bevorzugt 1 bis 69 Gew.-% und besonders bevorzugt 5 bis 60 Gew.-% Füllstoffe, Thixotropie-Hilfsmittel, Verzögerer und andere Hilfsstoffe,
(g) 20 bis 80 Gew.-%, bevorzugt 30 bis 75 Gew.-% und besonders bevorzugt 35 bis 70 Gew.-% übliche Weichmacher,
enthält.

Im weiteren wird Komponente I auch als Basispaste und Komponente II als Katalysatorpaste bezeichnet.

Als Bestandteil (a) werden mono-, di- oder multifunktionelle Vinylether verstanden, wie C3- bis C40-Alkylvinylether, bevorzugt C4- bis C30-Alkylvinylether, C8- bis C40-Arylvinylether, bevorzugt C8- bis C30-Arylvinylether, Aryl-alkylvinylether und cycloaliphatische Vinylether verstanden. Ebenso sind oligomere bzw. polymere Vinylether möglich, wie Polyethervinylether, Polyacrylatvinylether, Polyestervinylether, Polycarbonatvinylether, Polyimidvinylether, Polyamidvinylether, Phosphazenvinylether, Siloxanvinylether, Polyurethanvinylether. Vorteilhaft ist, wenn das Molekulargewicht der oligomeren bzw. polymeren Vinylether kleiner als 15.000 g/mol, bevorzugt kleiner als 10.000 g/mol ist. Weiterhin ist es für die Herstellung der Dentalmassen günstig, wenn die Vinylether bei Raumtemperatur als Flüssigkeit vorliegen. Natürlich können die Vinylether auch noch weitere funktionelle Gruppen, wie Hydroxygruppen, Epoxygruppen und (Meth)acryloxygruppen enthalten. Beispiele hierfür sind Di-methanol-cyclohexyl-monovinylether, Triethylenglycol-monovinylether-mono(meth)acrylsäureester und Cyclohexyl-3,4-epoxy-1-methylvinylether.

Besonders gut eignen sich Ethylenglycoldivinylether, Diethylenglycoldivinylether, 7,7,9-Trimethyl-4,13-dioxo-3,14,-dioxa-5,12-diazahexadecan-1,16-dioxydivinylether, Triethylenglycoldivinylether (BASF, Ludwigshafen), Isopropylvinylether, Tetraethylenglycoldivinylether, Tripropylenglycoldivinylether, 1,4-Di-methanol-cyclohexyl-divinylether (ISP, Guildford), Divinylether des Propandiols, Butandiols, Hexandiols, Octandiols, Nonandiols, Decandiols und Eicosandiols, Oktylvinylether, Oktadecylvinylether, Glycerintrivinylether, Pentaerythrit-tetravinylether, Bisphenol-A-divinylether, Divinylether des Bishydroxymethyltricyclo(5.2.1.0^{2,6})-decans und Divinylether des ethoxylierten und/oder propoxylierten Bisphenol-A oder Bisphenol-C.

Besonders gut eignen sich auch die Vinylether obiger Gruppe mit einer Viskosität kleiner 1 Pas bei 23°C und insbesondere kleiner 0,5 Pas bei 23°C, da sie den zusätzlichen Vorteil haben, die Viskosität der Dentalmassen herabzusetzen, wodurch das Ausbringen aus Kartuschen und ein automatisches Anmischen erleichtert wird.

Difunktionelle Vinylether sind allgemein besonders gut geeignet.

Ethylenisch ungesättigte Monomere der Komponente (b) sind monofunktionelle bzw. bi- oder höherfunktionelle ethylenisch ungesättigte Monomere, ausgenommen der Vinylether.

Unter dem Begriff ethylenisch ungesättigte Monomere im Sinne der vorliegenden Erfindung sind auch solche polymerisierbaren Verbindungen zu verstehen, die ein oligomeres bzw. polymeres Grundgerüst besitzen und mindestens eine ethylenisch ungesättigte Gruppe tragen. Diese ethylenisch ungesättigte Gruppe kann beispielsweise als Acrylat- und/oder Methacrylatgruppe vorliegen, die an das Grundgerüst kovalent gebunden ist. Das polymere Grundgerüst kann z.B. ein Polyethylenoxid, ein Polyester, ein Polyvinylesterderivat, ein Polyurethan, ein Polycarbonat, ein Polyalkohol, ein Polystyrol oder eine polymerisierte ethylenisch ungesättigte Verbindung sein. Das Molekulargewicht dieser Verbindungen sollte unter 25.000 g/mol, insbesondere unter 15.000 g/mol liegen.

Bevorzugte ethylenisch ungesättigte Monomere sind Methacrylat- und Acrylatmonomere, wie Methyl(meth)acrylat, n- oder i-Propyl(meth)acrylat, n-, i- oder tert.-Butyl(meth)acrylat und 2-Hydroxyl(meth)acrylat, 2-(Meth)acryloxy-tetrahydrofuran, 2-(((Alkylamino)-carbonyl)-oxy)ethyl-(meth)acrylate, Di(meth)acrylate des Propandiols, Butandiols, Hexandiols, Octandiols, Nonandiols, Decandiols und Eicosandiols, Tetrahydrofurfuryl(meth)acrylat, Di(meth)acrylate des Ethylenglycols, der Polyethylenglycole, der Polypropylenglycole, Di(meth)acrylate des ethoxylierten Bisphenol-A, z.B. 2,2'-Bis(4-(meth)acryloxy-tetraethoxyphenyl)propane, Urethan(meth)acrylate und (Meth)acryl-amide.

Ferner können als Monomere Ester der α-Cyanoacrylsäure, Crotonsäure, Zimtsäure und Sorbinsäure verwendet werden.

Weiterhin verwendbar sind die in der EP-A-0 235 826 genannten Methacrylsäureester, wie Triglykolsäure-bis[3[4]-methacryloxymethyl-8(9)-tricyclo[5.2.1.0^{2,6}]-decylmethylester.

Insbesondere geeignet sind 2,2-Bis-4(3-methacryloxy-2-hydroxypropoxy)phenylpropan (bis-GMA), 2,2-Bis-4(3-methacryloxypropoxy)phenylpropan, Triethylenglykoldimethacrylat (TEGDMA), 7,7,9-Trimethyl-4,13-dioxo-3,14,-dioxa-5,12-diazahexadecan-1,16-dioxy-dimethacrylat (UDMA), Urethan(meth)acrylate, und Di(meht)acrylate des Bishydroxymethyltricyclo(5.2.1.0^{2,6})-decans.

Diese ethylenisch ungesättigten Monomere können in den erfindungsgemäßen Dentalmassen entweder alleine oder in Kombination mit weiteren ethylenisch ungesättigten Monomeren eingesetzt werden.

Vorteilhaft für dieses Initiatorsystem hat sich der Einsatz von mehr als ca. 10 Gew.-% Urethan(meth)acrylaten als Monomer im Bestandteil (b) erwiesen, besonders wenn diese mehr als 50 Gew.-% von (b) ausmachen und insbesondere wenn (b) zu mehr als 80 Gew.-% aus Urethan(meth)acrylaten besteht. Selbstverständlich kann (b) auch zu 100 Gew.-% aus einem oder mehreren Urethan(meth)acrylaten bestehen.

Bei den Urethanmethacrylaten kann es sich beispielsweise um niedermolekulare Verbindungen, wie 7,7,9-Trimethyl-4,13-dioxo-3,14,-dioxa-5,12-diazahexadecan-1,16-dioxy-dimethacrylat und/oder oligomere bzw. polymere Verbindungen, wie Polyesterurethan(meth)acrylate, Polyetherurethan(meth)acrylate, Polycarbonaturethan(meth)acrylate und Poly(meth)acrylaturethan(meth)acrylate handeln. Vorzugsweise liegt das Molekulargewicht dieser Verbindungen kleiner 20.000 g/mol, besonderes kleiner 15.000 g/mol und insbesondere kleiner 10.000 g/mol.

Als Beschleuniger gemäss Bestandteil (c) sind Schwermetallverbindungen, insbesondere Metalle der Eisen- oder der Kupfergruppe, bevorzugt Kupfer- und Eisenkomplexe und insbesondere Kupferkomplexe geeignet. Das Schwermetall wird vorzugsweise in Form löslicher organischer Verbindungen eingesetzt. Geeignet sind beispielsweise Eisencarboxylate, Kupfercarboxylate, Eisenprocetonat, Kupferprocetonat, Kupfemaphthenat, Kupferacetat und Eisennaphthenat.

Außerdem können ionogen gebundene Halogene oder Pseudohalogene, z.B. Cl⁻-enthaltende Verbindungen, bevorzugt in Form von löslichen Salzen, insbesondere organische Ammoniumchloride oder Hydrochloride als zusätzliche Beschleuniger zugesetzt werden. Beispielsweise können Dibutylaminhydrochlorid, Tributylaminhydrochlorid, Tetrabutylammoniumchlorid, Triethylaminhydrochlorid, (β-Phenylethyl)-dibutyl-ammonium-chlorid und Polyethyleniminhydrochlorid eingesetzt werden.

Ferner kann die erfindungsgemäße polymerisierbare Dentalmasse als Bestandteil (d) und (f) übliche Füllstoffe für Dentalwerkstoffe, wie Glas- und Quarzpulver, Kieselgele, pyrogene hochdisperse Kieselsäuren, schwer lösliche Fluoride sowie Mischungen dieser Komponenten enthalten. Diese Füllstoffe können durch geeignete Zusätze, wie barium- und/oder strontiumhaltige Gläser, röntgenopak sein. Als Thixotropiemittel sind z.B. pyrogene hochdisperse Kieselsäuren geeignet. Weitere Hilfsstoffe sind beispielsweise Farbstoffe, Pigmente, Fliessverbesserer, polymere Verdickungsmittel oder Stabilisatoren. Zur Erhöhung der Flexibilität der Dentalmasse können auch lösliche organische Polymerisate, wie z.B. Polyvinylacetat sowie dessen Copolymere, zugesetzt werden.

Auch Christobalit, Calciumsilikat, Zirkoniumsilikat, Mondmorillonite, wie Bentonite, Zeolithe, einschließlich der Molekularsiebe, wie Natriumaluminiumsilikat, Metalloxidpulver, wie Aluminium- oder Zinkoxide bzw. deren Mischoxide, Bariumsulfat, Yttriumfluorid, Calciumcarbonat, Gips und Kunststoffpulver sind als Füllstoffe für die erfindungsgemäße Dentalmasse geeignet.

Die genannten Füllstoffe können auch durch z.B. eine Behandlung mit Organosilanen bzw -siloxanen oder durch die Veretherung von Hydroxylgruppen zu Alkoxygruppen hydrophobiert sein. Die Füllstoffe haben vorzugsweise eine Korngröße kleiner 0,100 mm, insbesondere kleiner 0,050 mm und ganz besonders kleiner 0,025 mm. Für besonders glatte Oberflächen und eine gute Polierbarkeit ist es vorteilhaft, Füllstoffe kleiner 0,005 mm einzusetzen.

Als Verzögerer sind die in der EP-B-0 374 824 beschriebenen Verbindungen geeignet.

Die Barbitursäurederivate und/oder Malonylsulfamide der Komponente (e) tragen alle mindestens ein acides Proton. Dieses befindet sich bei den Barbitursäurederivaten in Position 5 des Sechsrings. Es können beispielsweise alkyl-, aryl- und/oder cycloalkyl- substituierte Barbitursäurederivate und/oder Malonylsulfamide eingesetzt werden. Beispielsweise können folgende Verbindungen eingesetzt werden: 1,3,5- Trimethylbarbitursäure, 1,3,5-Triethylbarbitursäure, 1,3-Dimethyl-5-ethylbarbitursäure, 1,5-Dimethylbarbitursäure, 1-Methyl-5-ethylbarbitursäure, 1-Methyl-5-propylbarbitursäure, 5-Ethylbarbitursäure, 5-Propylbarbitursäure, 5-Butylbarbitursäure, 1-Benzyl-5-phenylbarbitursäure, 1-Cyclohexyl-5-ethylbarbitursäure und die in der DE-A-42 19 700 genannten Thiobarbitursäuren.

Gut geeignet sind auch die in der DT-C-14 95 520 beschriebenen Barbitursäuren und Barbitursäurederivate sowie die in der EP-A-0 059 451 genannten Malonylsulfamide. Bevorzugte Malonylsulfamide sind 2,6-Dimethyl-4-isobutylmalonylsulfamid, 2,6-Diisobutyl-4-propylmalonylsulfamid, 2,6-Dibutyl-4-propylmalonyl-sulfamid, 2,6-Dimethyl-4-ethylmalonylsulfamid, 2,6-Dioctyl-4-isobutylmalonylsulfamid.

Als Bestandteil (g) können übliche Weichmacher enthalten sein. Dies sind beispielweise Polyethylenglykolderivate, Polypropylenglycole, niedermolekulare Polyester, Dibutyl-, Dioctyl-, Dinonyl-, Diphenylphthalat, Di(iso-nonyladipat), Tricresylphosphat, Paraffinöle, Glycerintriacetat, Bisphenol-A-diacetat, und Siliconöle.

Das Mischungsverhältnis von Basis- zu Katalysatorpaste liegt vorzugsweise im Bereich von 1:1 bis 50:1, besonders 2:1 bis 20:1 und insbesondere 4:1 bis 10:1.

Die erfindungsgemäßen Dentalmassen sind insbesondere geeignet als Füllungsmaterialien, Stumpfaufbaumaterialien, Befestigungszemente, provisorische Kronen- und Brückenmaterialien, zahntechnische Werkstoffe, Modellmaterial oder zur Herstellung von Inlays, Onlays und Verblendschalen.

Erfindungsgemäß sind auch Behältnisse, enthaltend die zwei Komponenten der Dentalmassen, beispielsweise Kartuschen der Firma Mixpack, Rotkreuz, Schlauchbeutel der Firma ESPE, Seefeld und andere Kartuschen, Beutel, Tuben und Mischer.

### Beispiele

Aus den in Tabelle 1 und Tabelle 2 aufgeführten Bestandteilen werden jeweils 10g Katalysator- und 100 g Basispaste unter Vakuum geknetet. Diese werden in 10:1-Kartuschen der Firma Mixpack, Rotkreuz gefüllt. Zur Anwendung werden sie mittels eines Dispensers durch eine statische Mischvorrichtung gedrückt und dabei vermischt, so dass die Aushärtung innerhalb von einigen Minuten eintritt.

**Tabelle 1:**

| **Katalysator 1 mit Peroxid** | | |
|---|---|---|
| **Bestandteil** | **Rohstoff** | **Gew.-%** |
| (f) | Fluoroaluminosilicatglaspulver (∅<12µm) | 36 |
| (e) | 1-Benzyl-5-phenylbarbitursäure | 10 |
| Peroxid | 3,5,5-Trimethylhexansäure-tertiärbutylperester (TBPIN) | 4 |
| (g) | 2,2-Bis-4-(2-hydroxyethoxyphenyl)-propan-bis-acetat | 50 |

| **Katalysator 2 ohne Peroxid** | | |
|---|---|---|
| **Bestandteil** | **Rohstoff** | **Gew.-%** |
| (f) | Fluoroaluminosilicatglaspulver (∅<12µm) | 34 |
| (e) | 1-Benzyl-5-phenylbarbitursäure | 10 |
| (g) | 2,2-Bis-4-(2-hydroxyethoxyphenyl)-propan-bis-acetat | 56 |

**Tabelle 2:**

| **Basis 1 ohne Vinylether** | | |
|---|---|---|
| **Bestandteil** | **Rohstoff** | **Gew.-%** |
| (d) | Fluoroaluminosilicatglaspulver (∅<12µm) silanisiert mit Methacryloxypropyltrimethoxysilan | 25 |
| (d) | Mikrofeine Kieselsäure silanisiert (HDKH 2000, Wacker) | 7 |
| (c) | Bis-(1-phenylpentan-1,3-dionato)-kupfer(II) | 0,008 |
| (c) | (β-Phenylethyl)-dibutyl-ammonium-chlorid | 0,352 |
| (b) | UDMA | 60,84 |
| (b) | Triethylenglycoldimethacrylsäureester | 6,8 |

| **Basis 2 mit Vinylether (erfindungsgemäß)** | | |
|---|---|---|
| **Bestandteil** | **Rohstoff** | **Gew.-%** |
| (d) | Fluoroaluminosilicatglaspulver (∅<12µm) silanisiert mit Methacryloxypropyltrimethoxysilan | 25 |
| (d) | mikrofeine Kieselsäure silanisiert (HDKH 2000, Wacker) | 7 |
| (c) | Bis-(1-phenylpentan-1,3-dionato)-kupfer(II) | 0,008 |
| (c) | (β-Phenylethyl)-dibutyl-ammonium-chlorid | 0,352 |
| (b) | UDMA | 60,84 |
| (a) | 1,4-Di-methanol-cyclohexyl-divinylether | 6,8 |

### Mechanische Werte und Abbindezeiten:

**Tabelle 3:**

| **Messwert** | **Vergleichsbeispiel** | **Vergleichsbeispiel** | **Erfindungs- gemäßes Beispiel** |
|---|---|---|---|
| | Basis 1 / Katalysator 1 | Basis 1 / Katalysator 2 | Basis 2 / Katalysator 2 |
| Abbindeanfang [sec] | 35 | 75 | 40 |
| Abbindeende [sec] | 80 | 165 | 95 |
| Maximum der Abbinde-temperatur [°C] | 73 | 49 | 51 |
| Biege-E-Modul [MPa] | 1570 | 990 | 1620 |
| Abbindezeiten: Curometer (Fa. Wallace-Shawbury, England); | | | |
| E-Modul: 3-Punktbiegeversuch mit Biegestäbchen 4mm*6mm*25mm, Auflage im Abstand von 20mm, Belastung in der Mitte (Fa. Zwick, Deutschland); | | | |
| Temperaturmessung: Für die Temperaturmessung wurde ein Delrinzylinder mit Innenmaßen von 10 mm Durchmesser und 10 mm Höhe mit dem angemischten Material befüllt und das Maximum der Abbindetemperatur im Material gemessen. | | | |

In Tabelle 3 ist zu erkennen, wie mit der erfindungsgemäßen Basis 2, die Vinylether enthält, und dem erfindungsgemäßen Katalysator 2 ohne Peroxid bei niedriger Abbindetemperatur hohe mechanische Werte erzielt werden können.

## Patentansprüche

1. Polymerisierbare Masse, umfassend zwei Komponenten, wobei Komponente I:
(a) 0,1 bis 20 Gew.-% mindestens eines Vinylethers,
(b) 10 bis 89,9 Gew.-% mindestens eines ethylenisch ungesättigten Monomers, das kein Vinylether ist,
(c) 0,001 bis 5 Gew.-% mindestens eines Beschleunigers,
(d) 9,999 bis 89,899 Gew.-% Füllstoffe, Thixotropie-Hilfsmittel, Verzögerer und anderer Hilfsstoffe,
und
Komponente II:
(e) 0,1 bis 20 Gew.-% mindestens eines Barbitursäurederivates und/oder eines Malonylsulfamides, das die radikalische Polymerisation auslösen kann,
(f) 0 bis 89,9 Gew.-% Füllstoffe, Thixotropie-Hilfsmittel, Verzögerer und anderer Hilfsstoffe,
(g) 10 bis 80 Gew.-% übliche Weichmacher,
enthält.

2. Polymerisierbare Masse nach dem vorangehenden Anspruch, wobei Komponente (a) ausgewählt ist aus der Gruppe: Alkylvinylether, Arylvinylether, Aryl-alkylvinylether und cycloaliphatische Vinylether.

3. Polymerisierbare Masse nach dem vorangehenden Anspruch, wobei Komponente (a) ausgewählt ist aus der Gruppe: Polyethervinylether, Polyacrylatvinylether, Polyestervinylether, Polycarbonatvinylether, Polyimidvinylether, Polyamidvinylether, Phosphazenvinylether, Siloxanvinylether und Polyurethanvinylether.

4. Polymerisierbare Masse nach Anspruch 3, wobei Komponente (a) eine Molekulargewicht unter 15.000 g/mol aufweist.

5. Polymerisierbare Masse nach einem der vorangehenden Ansprüche, wobei Komponente (a) bei Raumtemperatur flüssig ist.

6. Polymerisierbare Masse nach einem der vorangehenden Ansprüche, wobei Komponente (a) noch zusätzliche funktionelle Gruppen trägt.

7. Polymerisierbare Masse nach einem der vorangehenden Ansprüche, wobei Komponente (a) mindestens difunktionell ist.

8. Verwendung der polymerisierbaren Massen nach einem der vorangehenden Ansprüche zur Herstellung von Dentalmassen im Dentalbereich.

9. Verwendung der polymerisierbaren Masse nach einem der Ansprüche 1 bis 7 zur Herstellung von Füllungsmaterial, Stumpfaufbaumaterial, Befestigungszement, provisorisches Kronen- und Brückenmaterial, zahntechnischen Werkstoff oder Inlays, Onlays, Verblendschalen, Modellmaterialien.

10. Behältnis, enthaltend mindestens eine polymerisierbare Masse nach einem der Ansprüche 1 bis 7.

11. Verwendung von Vinylethern in durch Initiatorsysteme auf Basis mindestens eins Barbitursäurederivats und/oder Malonylsulfamides, das die radikalische Polymerisation auslösen kann, polymerisierbaren Massen zur Steigerung der mechanischen Werte bei gleichzeitiger Erniedrigung des Maximums der Abbindetemperatur.

12. Gehärtete Masse, entstanden aus einer polymerisierbaren Masse nach einem der Ansprüche 1 bis 7, mit einem Biege-E-Modul von über 1.500 MPa.

## Claims

1. Polymerizable material comprising two components, where
component I comprises:
(a) from 0.1 to 20% by weight of at least one vinyl ether,
(b) from 10 to 89.9% by weight of at least one ethylenically unsaturated monomer which is not a vinyl ether,
(c) from 0.001 to 5% by weight of at least one accelerator,
(d) from 9.999 to 89.899% by weight of fillers, thixotropy assistants, retardants and other auxiliaries,
and
component II comprises
(e) from 0.1 to 20% by weight of at least one barbituric acid derivative and/or at least one malonylsulphamide which is able to initiate the free-radical polymerization, and
(f) from 0 to 89.9% by weight of fillers, thixotropy assistants, retardants and other auxiliaries,
(g) from 10 to 80% by weight of conventional plasticizers.

2. Polymerizable material according to the preceding claim, where component (a) is selected from the group consisting of: alkyl vinyl ethers, aryl vinyl ethers, aryl alkylvinyl ethers and cycloaliphatic vinyl ethers.

3. Polymerizable material according to the preceding claim, where component (a) is selected from the group consisting of: polyether vinyl ether, polyacrylate vinyl ether, polyester vinyl ether, polycarbonate vinyl ether, polyimide vinyl ether, polyamide vinyl ether, phosphazene vinyl ether, siloxane vinyl ether and polyurethane vinyl ether.

4. Polymerizable material according to Claim 3, where component (a) has a molecular weight of less than 15,000 g/mol.

5. Polymerizable material according to one of the preceding claims, where component (a) is liquid at room temperature.

6. Polymerizable material according to one of the preceding claims, where component (a) also carries additional functional groups.

7. Polymerizable material according to one of the preceding claims, where component (a) is at least difunctional.

8. Use of the polymerizable material according to one of the preceding claims for the production of dental materials in the dental sector.

9. Use of the polymerizable material according to one of Claims 1 to 7 for the production of filling material, core build-up material, dental cement, temporary crown and bridge material, material in dental technology or inlays, onlays, veneers and model materials.

10. Container containing at least one polymerizable material according to one of Claims 1 to 7.

11. Use of vinyl ethers in materials which can be polymerized by means of initiator systems based on at least one barbituric acid derivative and/or malonylsulphonamide which is able to initiate free-radical polymerization, for increasing the mechanical values at the same time as lowering the maximum setting temperature.

12. Cured material formed from a polymerizable material according to one of Claims 1 to 7, having a modulus of elasticity in bending of greater than 1500 MPa.

## Revendications

1. Matière polymérisable, comprenant deux composants, dans laquelle
le composant I contient :
(a) 0,1 à 20 % en poids d'au moins un vinyléther,
(b) 10 à 89,9 % en poids d'au moins un monomère éthyléniquement insaturé, qui n'est pas un vinyléther,
(c) 0,001 à 5 % en poids d'au moins un accélérateur,
(d) 9,999 à 89,899 % en poids de charges, d'adjuvant de thixotropie, de retardateur et autres adjuvants,
et le composant II contient :
(e) 0,1 à 20 % en poids d'au moins un dérivé d'acide barbiturique et/ou un malonylsulfamide, qui peut déclencher la polymérisation radicalaire,
(f) 0 à 89,9 % en poids de charges, d'adjuvant de thixotropie, de retardateur et autres adjuvants,
(g) 10 à 80 % en poids de plastifiant usuel.

2. Matière polymérisable selon la revendication précédente, dans laquelle le composant (a) est choisi dans le groupe : alkylvinyléther, arylvinyléther, aryl-alkylvinyléther et vinyléther cycloaliphatique.

3. Matière polymérisable selon la revendication précédente, dans laquelle le composant (a) est choisi dans le groupe : polyéthervinyléther, polyacrylate-vinyléther, polyestervinyléther, polycarbonate-vinyléther, polyimidevinyléther, polyamidevinyléther, phosphazènevinyléther, siloxanevinyléther et polyuréthanevinyléther.

4. Matière polymérisable selon la revendication 3, dans laquelle le composant (a) présente une masse moléculaire inférieure à 15 000 g/mole.

5. Matière polymérisable selon l'une quelconque des revendications précédentes, dans laquelle le composant (a) est liquide à température ambiante.

6. Matière polymérisable selon l'une quelconque des revendications précédentes, dans laquelle le composant (a) porte encore des groupes fonctionnels supplémentaires.

7. Matière polymérisable selon l'une quelconque des revendications précédentes, dans laquelle le composant (a) est au moins difonctionnel.

8. Utilisation des matières polymérisables selon l'une quelconque des revendications précédentes pour la préparation de matières dentaires dans le domaine dentaire.

9. Utilisation de la matière polymérisable selon l'une quelconque des revendications 1 à 7 pour la préparation de matière de plombage, matière de structure tronquée, ciment de fixation, matière provisoire pour couronnes et bridges, matériau de technique dentaire ou inlays, onlays, cuvettes de mélange et matériaux de moulage.

10. Récipient, contenant au moins une matière polymérisable selon l'une quelconque des revendications 1 à 7.

11. Utilisation de vinyléthers dans des matières polymérisables au moyen de système d'amorceur à base d'au moins un dérivé d'acide barbiturique et/ou de malonylsulfamide, qui peut déclencher la polymérisation radicalaire, pour augmenter les valeurs mécaniques en abaissant en même temps le maximum de la température de prise.

12. Matière durcie, produite à partir d'une matière polymérisable selon l'une quelconque des revendications 1 à 7, avec un module E en flexion supérieur à 1500 MPa.
